(19) Europäisches Patentamt
European Patent Office
Office européen des brevets

(11) EP 4 806 460 A1

(12) **EUROPEAN PATENT APPLICATION**
published in accordance with Art. 153(4) EPC

(43) Date of publication:
**16.09.2026 Bulletin 2026/38**

(21) Application number: **24888805.9**

(22) Date of filing: **08.11.2024**

(51) International Patent Classification (IPC):
***A61K 39/00*** *(2006.01)* ***A61P 11/02*** *(2006.01)*
***A61P 11/06*** *(2006.01)* ***A61P 17/04*** *(2006.01)*
***A61P 27/14*** *(2006.01)* ***A61P 37/04*** *(2006.01)*
***A61P 37/08*** *(2006.01)* ***C07K 7/00*** *(2006.01)*
***C07K 16/00*** *(2006.01)* ***C07K 19/00*** *(2006.01)*

(52) Cooperative Patent Classification (CPC):
**A61K 39/00; A61P 11/02; A61P 11/06; A61P 17/04; A61P 27/14; A61P 37/04; A61P 37/08; C07K 7/00; C07K 14/00; C07K 16/00; C07K 19/00**

(86) International application number:
**PCT/JP2024/039742**

(87) International publication number:
**WO 2025/100514 (15.05.2025 Gazette 2025/20)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **09.11.2023 JP 2023191836**

(71) Applicants:
- **Funpep Co., Ltd.**
  **Ibaraki-shi, Osaka 567-0085 (JP)**
- **The University of Osaka**
  **Osaka 565-0871 (JP)**

(72) Inventors:
- **KAWABATA, Sotaro**
  **Ibaraki-shi, Osaka 567-0085 (JP)**
- **SAKAGUCHI, Makoto**
  **Ibaraki-shi, Osaka 567-0085 (JP)**
- **NAKAGAMI, Hironori**
  **Suita-shi, Osaka 565-0871 (JP)**
- **HAYASHI, Hiroki**
  **Suita-shi, Osaka 565-0871 (JP)**
- **MORISHITA, Ryuichi**
  **Suita-shi, Osaka 565-0871 (JP)**

(74) Representative: **Weickmann & Weickmann PartmbB**
**Postfach 860 820**
**81635 München (DE)**

Remarks:
The complete document including Reference Table(s) and the Sequence Listing(s) can be downloaded from the EPO website

# (54) VACCINE COMPOSITION FOR INDUCING ANTI-IGE ANTIBODY

(57) The present invention provides a vaccine composition comprising a complex of a T cell receptor antigen peptide and a B cell receptor antigen peptide, and capable of inducing the production of an antibody against IgE, wherein the B cell receptor antigen peptide comprises the amino acid sequence shown in any of SEQ ID NOs: 20 - 22.

EP 4 806 460 A1

[Fig. 2]

120
100
80
60
40
20
0
% of Control
$10^0$
$10^1$
$10^2$
$10^3$
Purified IgG (ug/mL)
16
20
21
22
23
24

| SEQ ID NO: | hIgE neutralizing activity IC50 |
|---|---|
| 16 | NC |
| 20 | ++ |
| 21 | +++ |
| 22 | + |
| 23 | NC |
| 24 | NC |

NC: not calculable, +:10^4 digits µg/mL

++: 10^3 digits µg/mL, +++: 10^2 digits µg/mL

## Description

[Technical Field]

The present invention relates to a vaccine composition. More particularly, the present invention relates to a vaccine composition that can induce an antibody against IgE in a living body.

[Background Art]

IgE is a type of immunoglobulin family that mediates allergic response in type I allergic diseases (e.g., asthma, allergic rhinitis, urticaria, etc.). The mechanism by which IgE causes type I allergic diseases is briefly as follows: IgE binds to high-affinity IgE receptors (FcεR1) present on the cell surface of mast cells and basophils, when an allergen binds to the IgE and forms a crosslink, the mast cells and basophils release chemical mediators, including histamine. The released chemical mediators cause increased vasodilation and vascular permeability. As a result, various allergic symptoms occur.

As one of the therapeutic drugs for type I allergic diseases, Omalizumab (trade name Xolair (registered trademark)), which is an anti-IgE monoclonal antibody, is commercially available. Omalizumab specifically binds to and removes free IgE in the blood, thereby reducing the frequency of binding between FcεR1 on the surface of mast cells and IgE, thus suppressing activation of mast cells.

Omalizumab has a certain effect on severe asthma and is one of the effective therapeutic drugs for allergic diseases; however, it still has several aspects to be improved. For example, omalizumab is a humanized mouse monoclonal antibody, and immunological reactions cannot be completely avoided when used in human patients. In addition, antibody drugs are expensive, and the cost of treatment using omalizumab is said to be 15,000 to 44,000 US dollars per patient per year. Furthermore, antibody drugs have disadvantages such as limited administration methods. Given this background, the development of a safer and lower-cost treatment method for allergic diseases has been strongly demanded.

In order to overcome these disadvantages, the development of alternative means such as antigen peptides and the like that can induce anti-IgE antibodies when administered to the body is being actively pursued. For example, Patent Literature 1 discloses an IgE immunogen construct capable of inducing anti-IgE antibody. However, the development of IgE immunogen capable of inducing anti-IgE antibody efficiently is constantly desired.

[Citation List]

[Patent Literature]

[Patent Literature 1]
WO2010067286

[Summary of Invention]

[Technical Problem]

In view of the background described above, the present invention aims to provide a novel IgE immunogen that can induce anti-IgE antibodies with extremely high efficiency.

[Solution to Problem]

The present inventors have conducted intensive studies of the above-mentioned problem and found that a complex of a T cell receptor antigen peptide having a specific amino acid sequence and a B cell receptor antigen peptide having a specific amino acid sequence in Cε3 region in the Fc region of IgE can induce anti-IgE neutralizing antibodies extremely efficiently in vivo. The complex found by the present inventors had superior IgE immunogenicity and was able to efficiently induce antibodies with high IgE neutralizing activity.

The present inventors have conducted further studies based on such findings and completed the present invention.

That is, the present invention provides the following.

[1] A vaccine composition comprising a complex of a T cell receptor antigen peptide and a B cell receptor antigen peptide, and capable of inducing the production of an antibody against IgE, wherein the B cell receptor antigen peptide comprises the amino acid sequence shown in any of SEQ ID NOs: 20 - 22.

[2] The vaccine composition of [1], wherein the T cell receptor antigen peptide comprises the amino acid sequence shown in SEQ ID NO: 1.
[3] The vaccine composition of [1] or [2], wherein, in the complex, the C-terminus of the T cell receptor antigen peptide is bonded to the N-terminus of the B cell receptor antigen peptide.
[4] The vaccine composition of any of [1] to [3], wherein the T cell receptor antigen peptide and the B cell receptor antigen peptide are bonded via a linker.
[5] The vaccine composition of any of [1] to [4], for the treatment or prevention of a disease accompanied by excessive IgE secretion.
[6] The vaccine composition of [5], wherein the disease accompanied by excessive IgE secretion is at least one selected from the group consisting of asthma, allergic asthma, allergic rhinitis, conjunctivitis, eczema, urticaria, atopic dermatitis, and anaphylactic hypersensitivity.

[A-1] A method for treating or preventing a disease accompanied by excessive IgE secretion in a subject, comprising administering, to the subject, a vaccine composition comprising a complex of a T cell receptor antigen peptide and a B cell receptor antigen peptide, and capable of inducing the production of an antibody against IgE, wherein the B cell receptor antigen peptide comprises the amino acid sequence shown in any of SEQ ID NOs: 20 - 22.
[A-2] The method of [A-1], wherein the T cell receptor antigen peptide comprises the amino acid sequence shown in SEQ ID NO: 1.
[A-3] The method of [A-1] or [A-2], wherein, in the complex, the C-terminus of the T cell receptor antigen peptide is bonded to the N-terminus of the B cell receptor antigen peptide.
[A-4] The method of any of [A-1] to [A-3], wherein the T cell receptor antigen peptide and the B cell receptor antigen peptide are bonded via a linker.
[A-5] The method of any of [A-1] to [A-4], wherein the disease accompanied by excessive IgE secretion is at least one selected from the group consisting of asthma, allergic asthma, allergic rhinitis, conjunctivitis, eczema, urticaria, atopic dermatitis, and anaphylactic hypersensitivity.
[B-1] A vaccine composition for use in the treatment or prophylaxis of a disease accompanied by excessive IgE secretion, the composition comprising a complex of a T cell receptor antigen peptide and a B cell receptor antigen peptide, and capable of inducing the production of an antibody against IgE, wherein the B cell receptor antigen peptide comprises the amino acid sequence shown in any of SEQ ID NOs: 20 - 22.
[B-2] The vaccine composition for use of [B-1], wherein the T cell receptor antigen peptide comprises the amino acid sequence shown in SEQ ID NO: 1.
[B-3] The vaccine composition for use of [B-1] or [B-2], wherein, in the complex, the C-terminus of the T cell receptor antigen peptide is bonded to the N-terminus of the B cell receptor antigen peptide.
[B-4] The vaccine composition for use of any of [B-1] to [B-3], wherein the T cell receptor antigen peptide and the B cell receptor antigen peptide are bonded via a linker.
[B-5] The vaccine composition for use of any of [B-1] to [B-4], wherein the disease accompanied by excessive IgE secretion is at least one selected from the group consisting of asthma, allergic asthma, allergic rhinitis, conjunctivitis, eczema, urticaria, atopic dermatitis, and anaphylactic hypersensitivity.
[C-1] Use of a vaccine composition comprising a complex of a T cell receptor antigen peptide and a B cell receptor antigen peptide, and capable of inducing the production of an antibody against IgE in the production of a medicament for the treatment or prevention of a disease accompanied by excessive IgE secretion, wherein the B cell receptor antigen peptide comprises the amino acid sequence shown in any of SEQ ID NOs: 20 - 22.
[C-2] The use of [C-1], wherein the T cell receptor antigen peptide comprises the amino acid sequence shown in SEQ ID NO: 1.
[C-3] The use of [C-1] or [C-2], wherein, in the complex, the C-terminus of the T cell receptor antigen peptide is bonded to the N-terminus of the B cell receptor antigen peptide.
[C-4] The use of any of [C-1] to [C-3], wherein the T cell receptor antigen peptide and the B cell receptor antigen peptide are bonded via a linker.
[C-5] The use of any of [C-1] to [C-4], wherein the disease accompanied by excessive IgE secretion is at least one selected from the group consisting of asthma, allergic asthma, allergic rhinitis, conjunctivitis, eczema, urticaria, atopic dermatitis, and anaphylactic hypersensitivity.

[Advantageous Effects of Invention]

According to the present invention, production of an anti-IgE antibody in a living body can be induced very efficiently. Therefore, it becomes possible to treat and/or prevent diseases accompanied by excessive IgE secretion, such as type I allergic diseases, safely at a low cost.

[Brief Description of Drawings]

[Fig. 1]

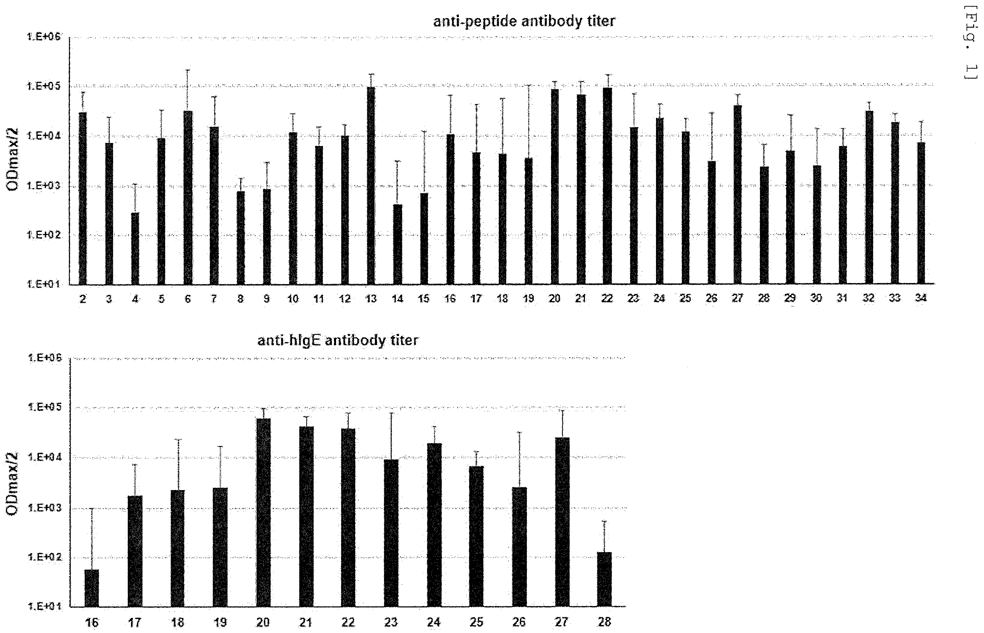

Fig. 1 is a diagram showing measurement results, by ELISA method, of antibody titer of antiserum derived from the mice administered with AJP001 conjugated peptide having the B cell epitope sequence of any of SEQ ID NOs: 2 - 34 against IgE synthetic peptide, and measurement results, by ELISA method, of antibody titer of antiserum derived from the mice administered with AJP001 conjugated peptide having the B cell epitope sequence of any of SEQ ID NOs: 16 - 28 against human IgE. (GMT±95%CI(N=4))
[Fig. 2]

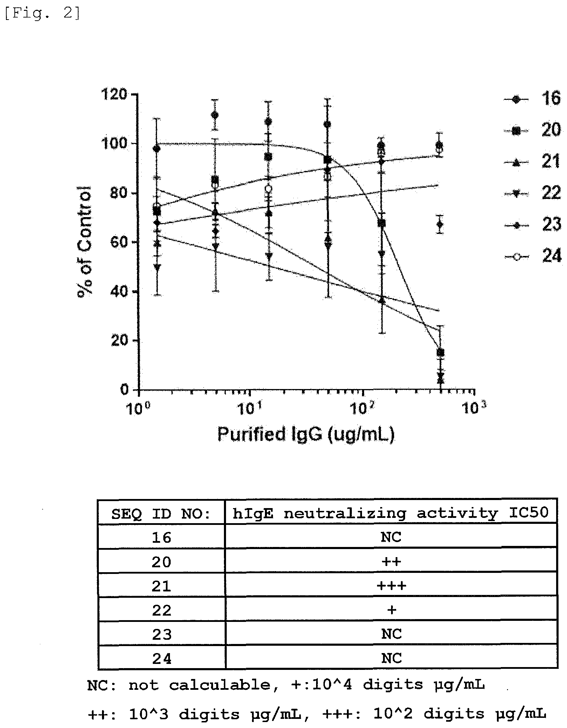

Fig. 2 is a diagram showing the evaluation results of neutralizing activity (inhibition of Luciferase expression, Mean ±SD (Triplicate)) of rat antiserum when administered with AJP001 conjugated peptide having the B cell epitope sequence of any of SEQ ID NOs: 16 and 20 - 24.
[Fig. 3]

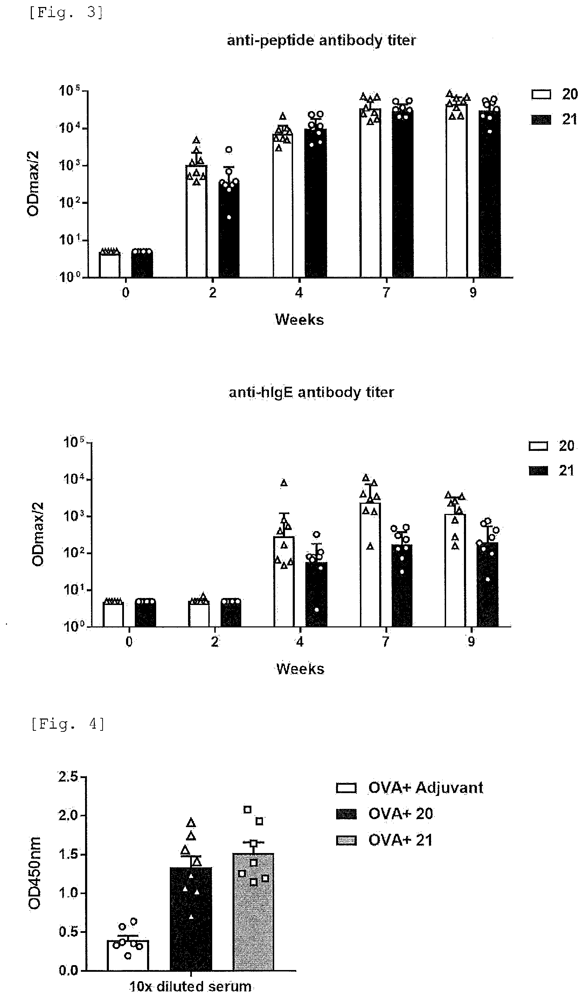

Fig. 3 is a diagram showing antibody titer of mouse serum of Humanized IgE/FcεR1 Tg mice administered with AJP001 conjugated peptide having the B cell epitope sequence of SEQ ID NO: 20 or 21 against each AJP001 conjugate peptide or human IgE. (GMT±95%CI, (N=8))
[Fig. 4]
Fig. 4 is a diagram showing concentration (Mean±SE (N=7 or 8)) of the complex in the serum of Humanized IgE/FcεR1 Tg mice when administered with AJP001 conjugated peptide having the B cell epitope sequence of SEQ ID NO: 20 or 21.
[Fig. 5]

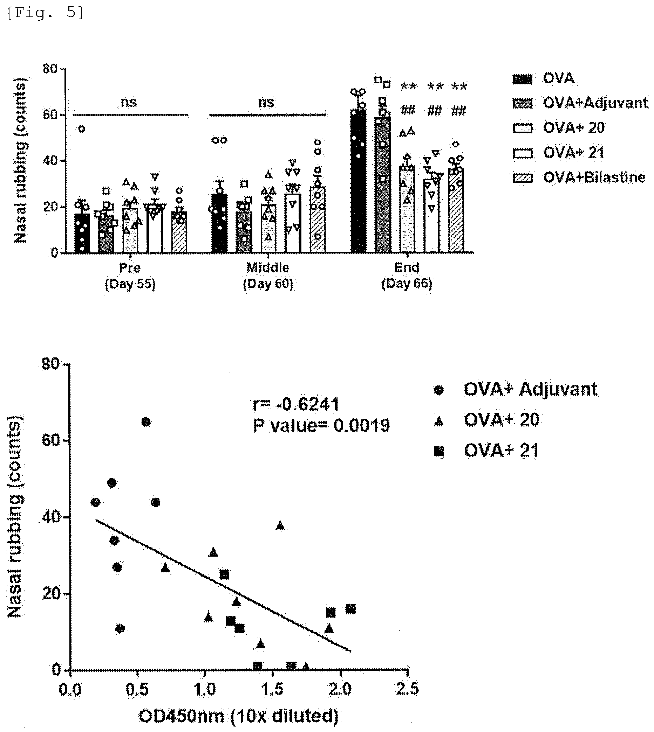

Fig. 5 is a diagram showing the number of nose-scratching behaviors (Mean±SE (N=8), **: p< 0.01 vs OVA group (t test), ##: p< 0.01 vs OVA+Adjuvant group (t test)) of Humanized IgE/FcεR1 Tg mice when administered with AJP001 conjugated peptide having the B cell epitope sequence of SEQ ID NO: 20 or 21, and the correlation between the number and complex concentration (Spearman's rank correlation coefficient).
[Fig. 6]

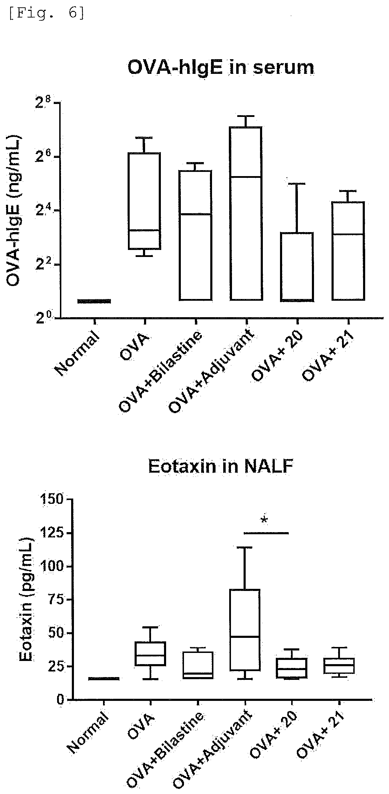

Fig. 6 is a diagram showing serum OVA-hIgE concentration (Min. to Max. (N=4-8)) and NALF Eotaxin concentration (Min. to Max. (N=4-8), *: P<0.05 v.s. OVA+Adjuvant group (t test)) in Humanized IgE/FcεR1 Tg mice when administered with AJP001 conjugated peptide having the B cell epitope sequence of SEQ ID NO: 20 or 21.
[Fig. 7]

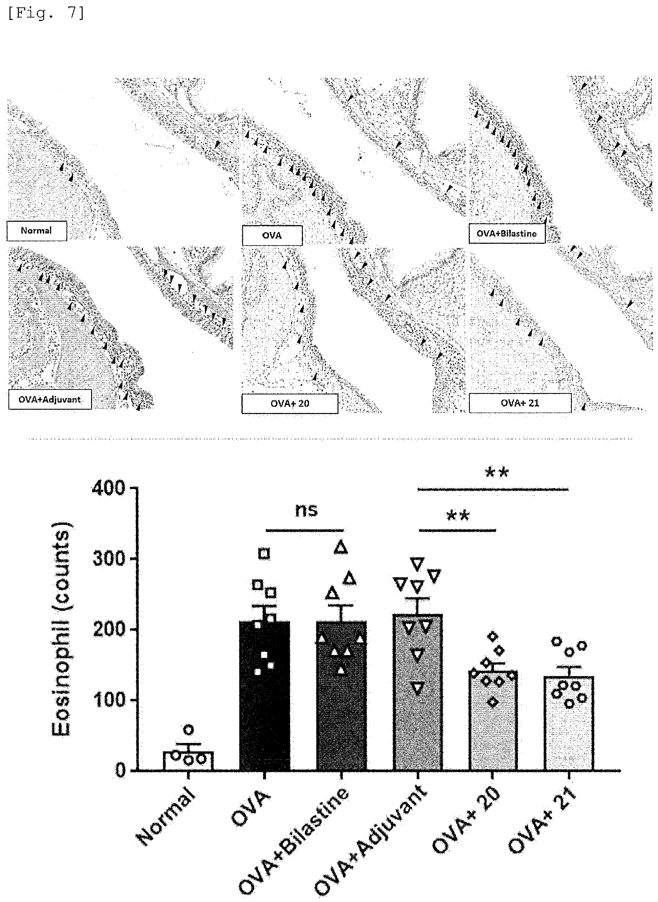

Fig. 7 is a diagram showing histopathological examination and eosinophil infiltration (Mean±SE (N=4-8), **:P<0.01 v.s. OVA+Adjuvant group (t test)) in the nasal cavity tissue of Humanized IgE/FcεR1 Tg mice administered with AJP001 conjugated peptide having the B cell epitope sequence of SEQ ID NO: 20 or 21.

[Description of Embodiments]

The present invention is described in detail below.

1. Vaccine composition

The present invention provides a vaccine composition containing a complex of a T cell receptor antigen peptide and a B cell receptor antigen peptide and capable of inducing the production of an antibody against IgE, wherein the B cell receptor antigen peptide comprises the amino acid sequence shown in any of SEQ ID NOs: 20 - 22 (hereinafter sometimes referred to as "the vaccine composition of the present invention").

The peptide in the present specification is described according to the common practice of peptide designation, wherein the left end indicates the N-terminus (amino terminus) and the right end indicates the C-terminus (carboxyl terminus). In the peptide complex contained as an active ingredient in the vaccine composition of the present invention, the C-terminus may be any of a carboxyl group (-COOH), carboxylate ($-COO^-$), amide ($-CONH_2$) and ester (-COOR).

Here, as R in the ester, a $C_{1-6}$ alkyl group, for example, methyl, ethyl, n-propyl, isopropyl and n-butyl; a $C_{3-8}$ cycloalkyl group, for example, cyclopentyl and cyclohexyl; a $C_{6-12}$ aryl group, for example, phenyl and α-naphthyl; a phenyl-$C_{1-2}$ alkyl group, for example, benzyl and phenethyl; a $C_{7-14}$ aralkyl group, for example, an α-naphthyl-$C_{1-2}$ alkyl group such as α-naphthylmethyl; a pivaloyloxymethyl group; and the like can be used.

When the peptide complex has a carboxyl group (or carboxylate) at a position other than the C-terminus, a peptide wherein the carboxyl group is amidated or esterified is also included in the peptide complex of the present invention. In this case, as the ester, for example, the above-described ester at the C terminus and the like are used.

Furthermore, the peptide complex also includes a peptide complex wherein the amino group of the N-terminal

amino acid residue is protected by a protecting group (e.g., $C_{1-6}$ acyl groups such as $C_{1-6}$ alkanoyls such as formyl group and acetyl group, and the like), a peptide complex wherein an amino group of the N-terminal amino acid residue is acetylated, a peptide complex wherein a substituent (e.g., -OH, -SH, amino group, imidazole group, indole group, guanidino group and the like) on a side chain of an amino acid in the molecule is protected by an appropriate protecting group (e.g., $C_{1-6}$ acyl groups such as $C_{1-6}$ alkanoyl groups such as formyl group and acetyl group, and the like), and the like. In one embodiment, in the peptide complex, an amino group of the N-terminal amino acid residue is acetylated and/or a C-terminal carboxyl group is amidated.

The peptide complex to be contained as the active ingredient in the vaccine composition of the present invention contains a B cell receptor antigen peptide in a part thereof. As used herein, the B cell receptor is a receptor expressed on the surface of B cells. Stimulated by an antigen peptide, the B cells proliferate and secrete the B cell receptor as an antibody against antigen peptide.

The B cell receptor antigen peptide in the vaccine composition of the present invention contains the amino acid sequence shown in any of SEQ ID NOs: 20 - 22. The amino acid sequences shown in SEQ ID NOs: 20 - 22 are specifically as follows:

GKPVNHSTRKEEKQRNGT (SEQ ID NO: 20)
SGKPVNHSTRKEEKQRNGT (SEQ ID NO: 21)
ASGKPVNHSTRKEEKQRNGT (SEQ ID NO: 22)

In one embodiment of the present invention, the B cell receptor antigen peptide may comprise or consist of the amino acid sequence shown in any of SEQ ID NOs: 20 - 22.

A part of the complex contained in the vaccine composition of the present invention is a T cell receptor antigen peptide. The T cell receptor antigen peptide is not particularly limited as long as it is an antigen peptide that forms a complex with MHC class II, is recognized by CD3/TCR complex and CD4, and transmits signals into CD3-positive cells. MHC class II includes, for example, HLA-DR, HLA-DQ, and HLA-DP in the case of human, and H-2A and H-2B in the case of mouse, each of which is a dimer composed of $\alpha$ chain and $\beta$ chain (e.g., HLA-DRA as $\alpha$ chain and HLA-DRB1 as $\beta$ chain for HLA-DR), preferably HLA-DR, HLA-DQ, or HLA-DP.

In the vaccine composition of the present invention, the T cell receptor antigen peptide is not particularly limited as long as it contains an epitope sequence that can be presented on MHC class II molecules of antigen-presenting cells to activate helper T cells. For example, AJP001 peptide (ELKLIFLHRLKRLRKRLKRK (SEQ ID NO: 1), see WO2016/047763 for the detail) developed by the present inventors, UBITh peptide (UBITh (registered trademark) 1: ISITEIKGVIVHRIE-TILF (SEQ ID NO: 35), UBITh (registered trademark) 2: KKKIITITRIITIITTID (SEQ ID NO: 36), UBITh (registered trademark) 3: ISISEIKGVIVHKIETILF (SEQ ID NO: 37), ISITEIRTVIVTRIETILF (SEQ ID NO: 38), see US patent no. 9,102,752 for the detail), Tetanous toxisoid peptide (TTaa830-843 peptide: QYIKANSKFIGITE (SEQ ID NO: 39)), and the like can be used. AJP001 induces the secretion of IL-$\beta$1 and IL-18 through activation of NLRP3 inflammasome, and can also activate the innate immune system by inducing the production of TNF-$\alpha$ and IL-6 through NF-$\kappa$B pathway. Thus, it is particularly preferred as a T cell receptor antigen peptide in the present invention.

In the vaccine composition of the present invention, a T cell receptor antigen peptide and a B cell receptor antigen peptide are bonded to form a complex. The bond may be a linkage between the terminus of one peptide chain and the terminus of the other peptide chain, or a linkage between the amino acid side chain of one peptide and the terminus of the other peptide chain, or a linkage between the both amino acid side chains, preferably a linkage between the terminus of one peptide chain and the terminus of the other peptide chain, more preferably a linkage between the C-terminus of one peptide chain and the N-terminus of the other peptide chain, further preferably a linkage between the C-terminus of T cell receptor antigen peptide and the N-terminus of B cell receptor antigen peptide. When the terminus of one peptide chain and the terminus of the other peptide chain are linked, the terminal amino acids of the both may be directly linked by a peptide bond or via a linker (also to be referred to as "spacer" in the present specification). The linker is not particularly limited as long as it can connect a T cell receptor antigen peptide and a B cell receptor antigen peptide, can be taken up into an antigen presenting cell, can liberate the helper T cell epitope in the T cell receptor antigen peptide, and can present it on MHC class II molecules. For example, amino acids other than $\alpha$-amino acid, such as $\varepsilon$-aminocaproic acid, $\beta$-aminoalanine, $\gamma$-aminobutyric acid, 7-aminoheptanoic acid, 12-aminolauric acid, p-aminobenzoic acid, and the like, can be used. In addition, L-amino acid (e.g., glutamic acid, cysteine, lysine) present in natural proteins and D-amino acids thereof can also be used. In one preferred embodiment, the amino acid linker is $\varepsilon$-aminocaproic acid. Alternatively, a peptide linker consisting of any 2 to 15 amino acids can also be used. Examples thereof include, but are not limited to, a G linker, which is a peptide linker composed of glycine (Gly) or methylated glycine (MeG), a GS linker, which is a peptide linker composed of Gly or MeG and Ser, and the like. In another embodiment, a PEG linker containing polyethylene glycol (PEG) or a derivative of polyethylene glycol can also be used. A PEG linker further containing one or more selected from glycine (Gly), serine (Ser), glutamic acid (Glu), arginine (Arg), and lysine (Lys) can also be used.

In one embodiment, a complex in which a B cell receptor antigen peptide and a T cell receptor antigen peptide are

linked may further contain additional amino acids. Addition of amino acids is acceptable as long as the desired effect of the vaccine composition of the present invention is obtained. While the amino acid sequence to be added is not particularly limited, for example, a tag that facilitates detection, purification and the like of the complex can be mentioned. Examples of the tag include Flag tag, histidine tag, c-Myc tag, HA tag, AU1 tag, GST tag, MBP tag, fluorescence protein tag (e.g., GFP, YFP, RFP, CFP, BFP etc.), immunoglobulin Fc tag, and the like. The position at which the amino acid sequence is added is not particularly limited, and is preferably the N-terminus and/or C-terminus of the complex.

In another embodiment, the complex in the vaccine composition of the present invention may be bonded to a functional molecule other than the aforementioned tag. Such functional molecules are not particularly limited as long as the vaccine composition of the present invention affords the desired effect. Examples of such functional molecules include, but are not limited to, molecules that have the function of suppressing the degradation of the complex when the vaccine composition of the present invention is administered in vivo.

A complex in the vaccine composition of the present invention can be produced by a solid phase synthesis process (Fmoc method and Boc method) or a liquid phase synthesis process, according to a known general protocol of peptide synthesis. When it is a complex in which a B cell receptor antigen peptide and a T cell receptor antigen peptide are linked directly or via an amino acid or peptide linker, the whole complex can be synthesized at once. Alternatively, a B cell receptor antigen peptide and a T cell receptor antigen peptide may be separately synthesized and thereafter the two peptides may be linked directly or via a linker.

In one embodiment, the peptide complex in the vaccine composition of the present invention may be conjugated with a carrier protein in order to increase the immunogenicity thereof. A carrier protein is generally a substance that binds to a molecule having no immunogenicity (hapten) due to its small molecular weight and imparts the immunogenicity, and is known in the technical field. Preferable examples of the carrier protein include bovine serum albumin (BSA), rabbit serum albumin (RSA), ovalbumin (OVA), keyhole-limpet hemocyanin (KLH), thyroglobulin (TG), diphtheria toxin made non-toxic by replacing some of its amino acids (CRM197), immunoglobulin, and the like. A carrier protein can be conjugated to the N-terminus or C-terminus of the complex in the vaccine composition of the present invention. Methods for conjugation include introducing a cysteine residue into the antigen peptide of the present invention, and binding the peptide to the amino group of the carrier protein via an SH group which is the side chain of cysteine (MBS method). In addition, amino groups such as $\varepsilon$ amino group, $\alpha$ amino group and the like of the lysine residue of a protein can also be bound to each other (glutaraldehyde method) to perform conjugation.

In one embodiment, the vaccine composition of the present invention may further contain a pharmaceutically acceptable adjuvant compatible with the active ingredient. Adjuvant is generally a substance that non-specifically potentiates an immune response of the host, and a number of adjuvants are known in the technical field. The adjuvant used for the vaccine composition of the present invention is not particularly limited as long as it can non-specifically potentiate immune responses. For example, aluminum salt (Alum), alum, CpG oligo deoxynucleotide, dsRNA, MONTANIDE (trademark, SEPPIC), squalane, saponin, and the like can be mentioned.

The vaccine composition of the present invention can be provided as a pharmaceutical composition containing a pharmaceutically acceptable carrier in addition to a complex of a T cell receptor antigen peptide and a B cell receptor antigen peptide.

The pharmaceutically acceptable carrier may be selected as appropriate according to the dosage form. Examples thereof include, but are not limited to, excipients such as sucrose, starch and the like, binders such as cellulose, methylcellulose and the like, disintegrants such as starch, carboxymethylcellulose and the like, lubricants such as magnesium stearate, and the like, aromatics such as citric acid, menthol and the like, preservatives such as sodium benzoate, sodium bisulfite and the like, stabilizers such as sodium citrate and the like, suspending agents such as methylcellulose, polyvinylpyrrolidone and the like, dispersing agents such as surfactant and the like, diluents such as water, saline and the like, base-wax and the like.

The vaccine composition of the present invention can be administered orally or parenterally to a mammal. Since a complex of a T cell receptor antigen peptide and a B cell receptor antigen peptide can be decomposed in the stomach, parenteral administration is preferable. A preparation preferable for oral administration includes liquid, capsule, sachet, tablet, suspension, emulsion and the like. A preparation preferable for parenteral administration (e.g., subcutaneous injection, muscular injection, topical injection, intraperitoneal administration and the like) includes aqueous and non-aqueous isotonic, aseptic injection liquid, which optionally contains antioxidant, buffer, bacteriostatic agent, isotonicity agent and the like. Also, an aqueous and non-aqueous aseptic suspending agent can be mentioned, and the agent optionally contains suspending agent, solubilizer, thickener, stabilizer, preservative and the like. Such preparation can be sealed in a unit dose or plural dose container such as ampoule or vial. In addition, the active ingredient and pharmaceutically acceptable carriers may be freeze-dried, and preserved in a form only requiring dissolution or suspending in a suitable aseptic vehicle immediately before use.

The content of the active ingredient (i.e., peptide complex) in a vaccine composition is, but not limited to, generally about 0.001 - 100 wt%, preferably about 0.05 - 99 wt%, further preferably 0.1 - 90 wt%, of the whole composition.

The subject of administration of the vaccine composition of the present invention is not particularly limited as long

as the subject may be affected with a disease in which the condition may worsen due to excessive IgE secretion (hereinafter sometimes referred to as "the disease accompanied by excessive IgE secretion"). Examples of the mammal include rodents such as mouse and the like, pets such as dog, cat and the like, domestic animals such as swine, horse, bovine and the like, primates such as human, monkey, orangutan, chimpanzee and the like, and the like, and human is particularly preferred.

The dose of the vaccine composition of the present invention varies depending on the recipient of administration, administration method, administration form and the like. Generally, 1 μg - 300,000 μg, preferably 20 μg - 30,000 μg, of a complex of a T cell receptor antigen peptide and a B cell receptor antigen peptide as the active ingredient is administered per dose to an adult 2 or 3 times for generally 4 weeks to 12 weeks. When the antibody titer falls, additional administration can be performed once each time.

Diseases that may be treated or prevented by the vaccine composition of the invention are the aforementioned disease accompanied by excessive IgE secretion. Examples of the disease include, but are not limited to, asthma, allergic asthma, allergic rhinitis, conjunctivitis, eczema, urticaria, atopic dermatitis, and anaphylactic hypersensitivity.

In one embodiment, the vaccine composition of the present invention may be used in combination with existing therapeutic agents for diseases accompanied by excessive IgE secretion. For example, in the treatment of allergic diseases, both the patients' QOL and treatment cost can be achieved by starting treatment with an immediately highly effective antibody drug (e.g., omalizumab, etc.) during the active stage of the disease and starting administration of the vaccine composition of the present invention during the remission stage. In addition, the vaccine composition of the present invention and existing therapeutic drugs can also be used simultaneously.

Note that the "treatment" of a disease in the present description can include not only cure of the disease but also remission of the disease and improvement of the severity of the disease.

Further, the "prevention" of a disease in the present description includes, in addition to prevention of the onset of the disease, delaying of the onset of the disease. In addition, the "prevention" of a disease in the present description can also include prevention of the recurrence of the disease after treatment, or delaying of the recurrence of the disease after treatment.

Further, the term "vaccine composition" in the present description can also be rephrased as "pharmaceutical composition" or "medicament".

2. Method for treating or preventing disease accompanied by excessive IgE secretion

The present invention also provides a method for treating or preventing a disease accompanied by excessive IgE secretion, including administering the vaccine composition of the present invention to a subject suffering or at risk of suffering from a disease accompanied by excessive IgE secretion (hereinafter sometimes referred to as "the method of the present invention").

In the method of the present invention, the subject of treatment or prevention, administration conditions for the vaccine composition of the present invention, and the like are the same as those described in "1. the vaccine composition of the present invention".

In one embodiment, the present invention provides a method for inducing the production of antibodies against IgE in a subject, comprising administering the vaccine composition of the present invention to the subject. In such method, the subject, administration conditions for the vaccine composition of the present invention, and the like are the same as those described in "1. the vaccine composition of the present invention".

The present invention is more specifically described in the following Examples, but the present invention is by no means limited by these examples.

[Example]

Synthesis of peptide (Fmoc method)

A protected peptide resin was synthesized by the Fmoc method according to the method described in 5th Edition, Jikken Kagaku Kouza 16, Synthesis of Organic Compounds IV and the like and using a fully automatic solid phase synthesizer. To the obtained protected peptide resin were added trifluoroacetic acid (TFA) and Scavenger (mixture of thioanisole, 2,2'-(ethylenedioxy)di-ethanethiol, m-cresol, triisopropyl silane, water, and the like), and a crude peptide was obtained by excising from the resin, followed by deprotection. The crude peptide was purified using a reverse phase HPLC column with gradient elution using a 0.1% TFA-$H_20$/$CH_3CN$ system. Fractions containing the target product were collected and lyophilized to obtain the peptide of interest.

HPLC analysis method for peptide

The purity of the synthesized peptide was measured using an HPLC device under the following analysis conditions.

HPLC model: Shimadzu Corporation LCLC-20ADXR
measurement wavelength: 220 nm
flow: 0.31 mL per minute
column: Inertsil ODS-3, 2.1m×250mm, 5 micron
column temperature: room temperature
mobile phase A: 0.1% trifluoroacetic acid aqueous solution
mobile phase B: acetonitrile
gradient conditions: linear gradient of mobile phase B
concentration from 5% to 80% in 30 min (5 → 80% buffer B in 30 min)

Mass spectrometry of peptide

The mass of the synthesized peptide was measured by MALDI-TOF-MS under the following analysis conditions. MALDI-TOF-MS model: Bruker autoflex speed matrix: 2,5-Dihydroxybenzoic acid
dissolve solution: mixed solution of 0.1% trifluoroacetic acid aqueous solution and acetonitrile

[Example 1] Rat immunogenicity evaluation test using AJP001 conjugated peptide (human IgE)

The epitope peptides of human IgE shown in Table 2 (SEQ ID NOs: 2 - 34) were selected as B cell antigens, and using T cell antigen AJP001 (Table 1, SEQ ID NO: 1) and ε-aminocaproic acid (sometimes referred to as "Ahx") as a spacer, conjugated complex (AJP001 conjugated peptide) was produced (production was outsourced to TORAY RESEARCH CENTER, INC. or PEPTIDE INSTITUTE, INC.). In the present specification, the AJP001 conjugated peptide whose B cell epitope has the amino acid sequence shown in SEQ ID NO: "X" is to be referred to as "AJP001 conjugated peptide (SEQ ID NO: X)" and the like.

[Table 1]

| SEQ ID NO | **amino acid sequence** |
|---|---|
| 1 | ELKLIFLHRLKRLRKRLKRK |

[Table 2]

| SEQ ID NO | amino acid position | **B cell epitope sequence** | **mass* (MW)** | **purity* (%)** |
|---|---|---|---|---|
| 2 | 203-216 | EDSTKKCADSNPRG | 4287.42 | 97.38 |
| 3 | 204-217 | DSTKKCADSNPRGV | 4257.41 | 97.11 |
| 4 | 205-218 | STKKCADSNPRGVS | 4229.34 | 96.77 |
| 5 | 199-216 | GHTFEDSTKKCADSNPRG | 4732.57 | 96.16 |
| 6 | 200-217 | HTFEDSTKKCADSNPRGV | 4774.64 | 96.42 |
| 7 | 201-218 | TFEDSTKKCADSNPRGVS | 4724.58 | 96.79 |
| 8 | 233-246 | KSPTITCLVVDLAP | 4236.52 | 95.07 |
| 9 | 234-247 | SPTITCLVVDLAPS | 4195.41 | 92.56 |
| 10 | 235-248 | PTITCLVVDLAPSK | 4236.70 | 97.19 |
| 11 | 229-246 | LFIRKSPTITCLVVDLAP | 4769.03 | 90.82 |
| 12 | 230-247 | FIRKSPTITCLVVDLAPS | 4742.65 | 96.08 |
| 13 | 231-248 | IRKSPTITCLVVDLAPSK | 4723.65 | 95.85 |
| 14 | 265-278 | HSTRKEEKQRNGTL | 4464.04 | 97.91 |
| 15 | 266-279 | STRKEEKQRNGTLT | 4428.00 | 96.37 |

(continued)

| SEQ ID NO | amino acid position | **B cell epitope sequence** | **mass* (MW)** | **purity* (%)** |
|---|---|---|---|---|
| 16 | 264-277 | NHSTRKEEKQRNGT | 4464.49 | 95.03 |
| 17 | 263-277 | VNHSTRKEEKQRNGT | 4566.21 | 98.39 |
| 18 | 262-277 | PVNHSTRKEEKQRNGT | 4663.56 | 97.92 |
| 19 | 261-277 | KPVNHSTRKEEKQRNGT | 4791.62 | 98.55 |
| 20 | 260-277 | GKPVNHSTRKEEKQRNGT | 4848.92 | 98.20 |
| 21 | 259-277 | SGKPVNHSTRKEEKQRNGT | 4935.71 | 95.70 |
| 22 | 258-277 | ASGKPVNHSTRKEEKQRNGT | 5007.26 | 97.43 |
| 23 | 255-277 | WSRASGKPVNHSTRKEEKQRNGT | 5436.47 | 95.22 |
| 24 | 253-277 | LTWSRASGKPVNHSTRKEEKQRNGT | 5650.51 | 96.44 |
| 25 | 250-277 | TVNLTWSRASGKPVNHSTRKEEKQRNGT | 5965.08 | 98.50 |
| 26 | 248-277 | KGTVNLTWSRASGKPVNHSTRKEEKQRNGT | 6150.54 | 98.24 |
| 27 | 261-278 | KPVNHSTRKEEKQRNGTL | 4904.75 | 98.33 |
| 28 | 262-279 | PVNHSTRKEEKQRNGTLT | 4877.67 | 98.36 |
| 29 | 295-308 | ETYQCRVTHPHLPR | 4516.67 | 97.60 |
| 30 | 296-309 | TYQCRVTHPHLPRA | 4458.71 | 98.38 |
| 31 | 297-310 | YQCRVTHPHLPRAL | 4470.93 | 98.31 |
| 32 | 291-308 | WIEGETYQCRVTHPHLPR | 5005.20 | 96.24 |
| 33 | 292-309 | IEGETYQCRVTHPHLPRA | 4889.60 | 98.25 |
| 34 | 293-310 | EGETYQCRVTHPHLPRAL | 4889.77 | 97.67 |
| * property value of each AJP001-Ahx-B cell epitope peptide conjugate obtained by chemical synthesis | | | | |

AJP001 conjugated peptide having one of the B cell epitope sequences of SEQ ID NOs: 2 - 34 was dissolved in physiological saline, mixed with 2% Alhydrogel (invivogen, 0.3 mg/body) and K3 Et-Free (GeneDesign, Inc., 0.1 mg/body), and subcutaneously administered three times to JCL:Wistar rats (female, 7-week-old, N=4) at 0.5 mg/body at 2-week intervals. Blood was collected before administration and 6 weeks after the initial administration, and the antibody titer against each epitope sequence and human IgE were measured by the ELISA method. Specifically, a 96-well plate immobilized with an epitope peptide dissolved at 10 μg/mL in carbonate buffer was blocked with 5% skim milk/PBS, and then serum serially diluted with 5% skim milk/PBS was added, and the mixture was left standing at 4°C overnight. After washing the wells with PBS-T, HRP-labeled anti-rat IgG antibody (BETHYL) diluted with 5% skim milk/PBS was added and the mixture was shaken at room temperature for 3 hr. After washing the wells with PBS-T, TMB solution (SIGMA) was added, the wells were allowed to stand for 30 min in the dark, the reaction was stopped by adding 0.1M $H_2SO_4$ (KANTO KAGAKU), and the absorbance at 450 nm was measured using a plate reader. The serum dilution rate that gave 1/2 (OD=1.75) of the maximum absorbance was defined as the antibody titer, and the geometric mean titer:GMT was calculated from the individual values. The figure shows the geometric mean antibody titer (GMT) ± 95% confidence interval (95% CI). Antibody titers against human IgE were measured by the ELISA method in the same manner as described above, using human IgE full length protein (abcam) as the immobilized antigen.

Antibody titer against each AJP001 conjugated peptide and human IgE are shown in Fig. 1. As a result, a significant increase in human IgE antibody titer was confirmed in the antiserum of rat administered with AJP001 conjugated peptide having the B cell epitope sequences of SEQ ID NOs: 17 - 27.

[Example 2] Evaluation of neutralizing activity of AJP001 conjugated peptide (human IgE) using rat antiserum

The neutralizing activity of anti-human IgE antibody produced by the AJP001 conjugated peptide was evaluated using serum at 6 weeks after the initial administration in Example 1. Stable expression cell line established by introducing human FcεRIα and NFAT-RE-Luciferase genes into RBL-2H3 cells (RBL-2H3/FCER1A+NFAT NLuc cells) was used. When human IgE is added to these cells, they bind to FcεRIα, then stimulation with an anti-human IgE antibody activates NFAT, and reporter gene (Luciferase) is expressed. Inhibition of Luciferase expression upon human IgE stimulation was

used as an indicator for evaluation. Specifically, for SEQ ID NOs. 16 and 20-24, pooled serum (400 μL/individual, N=4) was prepared, after an inactivation treatment (56°C, 30 min reaction), an equal volume of ammonium sulfate (FUJIFILM Wako Pure Chemical Corporation) was added, and the mixture was reacted at room temperature for 30 min while mixing by inversion. Thereafter, the solution was centrifuged at 4°C, 3000xg for 20 min, the precipitate was dissolved in PBS, and serum IgG antibodies (including anti-IgE antibodies) were purified using Protein G HP spin trap (Cytiva), Ab buffer kit (Cytiva), Amicon ultra-0.5 centrifugal filter devices (100K) (Millipore). Then, purified IgG (final concentration 1.2-500 μg/mL) and human IgE (final concentration 0.6 μg/mL) were added to culture medium (1% P/S, 400 μg/mL G418, 500 μg/mL Hygromycin B, 10% FBS-containing DMEM) and reacted at 37°C for 2 hr. Then, it was added at 50 μL/well to cells seeded on 96-Well white plate (ThermoFisher) (1.0 × 10^5 cells/50 μL/well) and reacted at 37°C for 24 hr. An untreated well and a control well to which human IgE alone was added were provided. After 24 hr, the cells were washed with maintenance medium, and anti-human IgE antibody (final concentration 1 μg/mL, AQI) was added and reacted at 37°C for 4 hr. Thereafter, NanoGlo Luciferase Assay substrate was added, and reacted for 3 min, and the luminescence value was measured using a luminescence plate reader. The proportion (%) of the luminescence value relative to the control well was calculated using the following formula, from the luminescence values of the untreated well (A), the control well (B), and the well of purified IgG derived from rat antiserum administered with each AJP001 conjugate peptide (C).

Based on the added concentration of purified IgG, a regression equation was calculated using a 4-parameter logistic model, and the 50% inhibitory concentration (IC50) was calculated from the regression equation. The neutralizing activity was expressed in steps from the IC50 concentration (μg/mL) (+: 10^4 digits, ++: 10^3 digits, +++: 10^2 digits, NC: when not calculable).

Proportion (%) of luminescence value relative to control well=100×[(C-A)/(B-A)]

The neutralizing activity against human IgE is shown in Fig. 2. As a result, neutralizing activity (inhibition of Luciferase expression) was confirmed in rat antiserum administered with AJP001 conjugate peptides having B cell epitope sequences of SEQ ID NOs: 20, 21, and 22.

<u>[Example 3] Efficacy evaluation test of AJP001 conjugate peptide (human IgE) in OVA-induced allergic rhinitis model using Humanized IgE/FcεR1 Tg mice</u>

The AJP001 conjugate peptide, having the B cell epitope sequence of SEQ ID NO: 20 or 21, was dissolved in saline, mixed with 2% Alhydrogel (invivogen, 0.3 mg/body) and K3 Et-Free (Genedesign, 0.1 mg/body), and then subcutaneously administered to Humanized IgE/FcεR1 Tg mice (female, 10 weeks old, N=8/group) at 0.5 mg/body three times at 2-week intervals (Days 0, 14, and 28). The positive control substance, an antihistamine drug (bilastine, Taiho Pharmaceutical Co., Ltd.), was orally administered at 50 mg/kg one hr before OVA administration on Days 56-66. In addition, an OVA+adjuvant group was established as a control group for the SEQ ID NO: 20 or 21 administration group, and an OVA group was established as a control group for the bilastine administration group. The pathology model was created by administering OVA (containing Alum) at 100 μg/body (intraperitoneally) on Day 35 and 50 μg/body (subcutaneously) on Day 49, followed by transnasal administration of 50 μg of OVA on Days 56-66. Under these test conditions, serum (Days 0, 14, 28, 35, 49, and 67) was collected, and antibody titers against each AJP001 conjugate peptide and human IgE, serum human IgE/anti-human IgE antibody complex concentrations, and serum OVA-specific human IgE concentrations were measured using ELISA method. In addition, video recording was performed 1 hr after transnasal administration on Days 55, 60, and 66, and the number of nose-scratching behaviors was evaluated. After euthanasia on Day 67, nasal lavage fluid (NALF) was collected, and the concentration of Eotaxin in the NALF was measured using ELISA method. Furthermore, after collecting nasal tissue, Luna-stained specimens were prepared, and the number of eosinophil infiltrations was measured. Antibody titers were measured using the same procedure as in Example 1, serum OVA-specific human IgE concentration was measured using Human Ovalbumin Specific IgE ELISA Kit (Finetest), and NALF Eotaxin concentration was measured using Mouse CCL11/Eotaxin immunoassay kit (R&D).

The antibody titers against each AJP001 conjugate peptide and human IgE are shown in Fig. 3, the serum complex concentration is shown in Fig. 4, the correlation between the number of nose-scratching behaviors and complex concentration is shown in Fig. 5, the serum OVA-hIgE concentration and NALF Eotaxin concentration are shown in Fig. 6, and the histopathological examination and the degree of eosinophil infiltration are shown in Fig. 7.

As a result, in the SEQ ID NO: 20 or 21 administration group, antibody titers increased over time, and the number of nose scratches and eosinophil infiltration in the nasal cavity tissue were significantly suppressed. Also, Eotaxin concentration in NALF and serum OVA-specific human IgE concentration showed a tendency toward lower values. From these results, it was suggested that the antibodies induced in the SEQ ID NO: 20 or 21 administration group can suppress rhinitis symptoms by binding to human IgE and inhibiting IgE/FcεR1 binding. On the other hand, the antihistamine drug (bilastine) showed effectiveness comparable to the SEQ ID NO: 20 or 21 administration group in the number of nose

scratches, but other parameters did not change. Therefrom it was suggested that the present IgE antibody-inducing peptide and the antihistamine drug suppress rhinitis symptoms through different action mechanisms.

[Industrial Applicability]

According to the present invention, a peptide vaccine for the treatment and/or prevention of diseases accompanied by excessive IgE secretion can be produced at a low cost. Therefore, the present invention is extremely useful in the field of manufacture of pharmaceutical products.

This application is based on a patent application No. 2023-191836 filed in Japan (filing date: November 9, 2023), the contents of which are incorporated in full herein.

[Sequence Listing]

## Claims

1. A vaccine composition comprising a complex of a T cell receptor antigen peptide and a B cell receptor antigen peptide, and capable of inducing the production of an antibody against IgE, wherein the B cell receptor antigen peptide comprises the amino acid sequence shown in any of SEQ ID NOs: 20 - 22.

2. The vaccine composition according to claim 1, wherein the T cell receptor antigen peptide comprises the amino acid sequence shown in SEQ ID NO: 1.

3. The vaccine composition according to claim 1 or 2, wherein, in the complex, the C-terminus of the T cell receptor antigen peptide is bonded to the N-terminus of the B cell receptor antigen peptide.

4. The vaccine composition according to claim 1 or 2, wherein the T cell receptor antigen peptide and the B cell receptor antigen peptide are bonded via a linker.

5. The vaccine composition according to claim 1 or 2, for the treatment or prevention of a disease accompanied by excessive IgE secretion.

6. The vaccine composition according to claim 5, wherein the disease accompanied by excessive IgE secretion is at least one selected from the group consisting of asthma, allergic asthma, allergic rhinitis, conjunctivitis, eczema, urticaria, atopic dermatitis, and anaphylactic hypersensitivity.

[Fig. 1]

anti-peptide antibody titer
ODmax/2
1.E+06
1.E+05
1.E+04
1.E+03
1.E+02
1.E+01
2 3 4 5 6 7 8 9 10 11 12 13 14 15 16 17 18 19 20 21 22 23 24 25 26 27 28 29 30 31 32 33 34
anti-hIgE antibody titer
ODmax/2
1.E+06
1.E+05
1.E+04
1.E+03
1.E+02
1.E+01
16 17 18 19 20 21 22 23 24 25 26 27 28

[Fig. 2]

120
100
80
60
40
20
0
% of Control
$10^0$
$10^1$
$10^2$
$10^3$
Purified IgG (ug/mL)
16
20
21
22
23
24

| SEQ ID NO: | hIgE neutralizing activity IC50 |
|---|---|
| 16 | NC |
| 20 | ++ |
| 21 | +++ |
| 22 | + |
| 23 | NC |
| 24 | NC |

NC: not calculable, +:10^4 digits µg/mL

++: 10^3 digits µg/mL, +++: 10^2 digits µg/mL

[Fig. 3]

anti-peptide antibody titer
ODmax/2
10⁵
10⁴
10³
10²
10¹
10⁰
0
2
4
7
9
Weeks
20
21

anti-hIgE antibody titer
ODmax/2
10⁵
10⁴
10³
10²
10¹
10⁰
0
2
4
7
9
Weeks
20
21

[Fig. 4]

OD450nm
2.5
2.0
1.5
1.0
0.5
0.0
10x diluted serum
OVA+ Adjuvant
OVA+ 20
OVA+ 21

[Fig. 5]

Nasal rubbing (counts)
80
60
40
20
0
ns
ns
** ** **
## ## ##
Pre
(Day 55)
Middle
(Day 60)
End
(Day 66)
OVA
OVA+Adjuvant
OVA+ 20
OVA+ 21
OVA+Bilastine

Nasal rubbing (counts)
80
60
40
20
0
0.0
0.5
1.0
1.5
2.0
2.5
OD450nm (10x diluted)
r= -0.6241
P value= 0.0019
OVA+ Adjuvant
OVA+ 20
OVA+ 21

[Fig. 6]

OVA-hIgE in serum
OVA-hIgE (ng/mL)
$2^8$
$2^6$
$2^4$
$2^2$
$2^0$
Normal
OVA
OVA+Bilastine
OVA+Adjuvant
OVA+ 20
OVA+ 21

Eotaxin in NALF
Eotaxin (pg/mL)
150
125
100
75
50
25
0
*
Normal
OVA
OVA+Bilastine
OVA+Adjuvant
OVA+ 20
OVA+ 21

[Fig. 7]

Normal
OVA
OVA+Bilastine
OVA+Adjuvant
OVA+ 20
OVA+ 21

Eosinophil (counts)
400
300
200
100
0
ns
**
**
Normal
OVA
OVA+Bilastine
OVA+Adjuvant
OVA+ 20
OVA+ 21

**INTERNATIONAL SEARCH REPORT**

International application No.
**PCT/JP2024/039742**

**A. CLASSIFICATION OF SUBJECT MATTER**

***A61K 39/00***(2006.01)i; ***A61P 11/02***(2006.01)i; ***A61P 11/06***(2006.01)i; ***A61P 17/04***(2006.01)i; ***A61P 27/14***(2006.01)i; ***A61P 37/04***(2006.01)i; ***A61P 37/08***(2006.01)i; ***C07K 7/00***(2006.01)i; ***C07K 16/00***(2006.01)i; ***C07K 19/00***(2006.01)i
FI: A61K39/00 H ZNA; A61P37/04; A61P11/06; A61P11/02; A61P27/14; A61P17/04; A61P37/08; C07K7/00; C07K19/00; C07K16/00

According to International Patent Classification (IPC) or to both national classification and IPC

**B. FIELDS SEARCHED**

Minimum documentation searched (classification system followed by classification symbols)

A61K39/00; A61P11/02; A61P11/06; A61P17/04; A61P27/14; A61P37/04; A61P37/08; C07K7/00; C07K16/00; C07K19/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| A | WO 2017/164409 A1 (OSAKA UNIVERSITY) 28 September 2017 (2017-09-28) in particular, claims, examples | 1-6 |
| A | JP 2002-537403 A (SMITHKLINE BEECHAM BIOLOGICALS S.A.) 05 November 2002 (2002-11-05) in particular, claims, examples, SEQ ID NO: 1 | 1-6 |
| A | JP 2004-514655 A (GLAXOSMITHKLINE BIOLOGICALS S. A.) 20 May 2004 (2004-05-20) in particular, claims, examples, SEQ ID NO: 60 | 1-6 |

☐ Further documents are listed in the continuation of Box C.  ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **27 November 2024** | **10 December 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**

International application No.
**PCT/JP2024/039742**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/JP2024/039742**

| Patent document cited in search report | Publication date (day/month/year) | Patent family member(s) | Publication date (day/month/year) |
|---|---|---|---|
| WO 2017/164409 A1 | 28 September 2017 | US 2019/0105388 A1<br>in particular, claims, examples<br>US 2021/0205446 A1<br>EP 3434279 A1<br>EP 4194007 A1<br>CN 108883166 A<br>KR 10-2018-0123064 A<br>JP 2021-183638 A<br>JP 2023-105112 A | |
| JP 2002-537403 A | 05 November 2002 | US 2005/0152892 A1<br>in particular, claims, examples, SEQ ID NO: 1<br>US 2003/0147906 A1<br>WO 2000/050461 A1<br>EP 1155038 A1<br>KR 10-2002-0007314 A<br>CN 1520423 A | |
| JP 2004-514655 A | 20 May 2004 | US 2004/0030106 A1<br>in particular, claims, examples, SEQ ID NO: 60<br>WO 2002/016409 A2<br>EP 1311536 A2<br>KR 10-2003-0062405 A<br>CN 1471539 A | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2010067286 A
- WO 2016047763 A
- US 9102752 B
- JP 2023191836 A